Europäisches Patentamt

European Patent Office

Office européen des brevets

(19)

(11) Veröffentlichungsnummer: **0 519 365 A1**

# EUROPÄISCHE PATENTANMELDUNG

(12)

(21) Anmeldenummer: **92110021.0**

(22) Anmeldetag: **13.06.92**

(51) Int. Cl.5: **A61K 9/20**, A61K 9/24,
A61K 9/28, A61K 9/32,
A61K 9/54, A61K 31/44

(30) Priorität: **17.06.91 CH 1788/91**

(43) Veröffentlichungstag der Anmeldung:
**23.12.92 Patentblatt 92/52**

(84) Benannte Vertragsstaaten:
**PT**

(71) Anmelder: **Byk Gulden Lomberg Chemische
Fabrik GmbH
Byk-Gulden-Strasse 2
W-7750 Konstanz(DE)**

(72) Erfinder: **Dietrich, Rango, Dr.
Im Tiergarten 16
W-7750 Konstanz 16(DE)**
Erfinder: **Ney, Hartmut
Peter-Thumb-Strasse 46
W-7750 Konstanz(DE)**

(54) **Pantoprazol enthaltende orale Darreichungsformen.**

(57) Die Erfindung betrifft orale Darreichungsformen für Pantoprazol, die aus einem Kern, einer Zwischenschicht und einer magensaftresistenten äußeren Schicht bestehen.

EP 0 519 365 A1

Stand der Technik

In der europäischen Patentanmeldung EP-A-244 380 werden orale Darreichungsformen für säurelabile Wirkstoffe aus der Klasse der $H^+/K^+$-ATPase-Hemmer mit Pyridylmethylsulfinyl-1H-benzimidazol-Struktur beschrieben, die einen Kern, eine Zwischenschicht und eine magensaftresistente äußere Schicht aufweisen. In der europäischen Patentanmeldung EP-A-247 983 werden die in der EP-A-244 380 offenbarten Formulierungen im Zusammenhang mit dem $H^+/K^+$-ATPase-Hemmer Omeprazol beschrieben und beansprucht.

Bei den in den europäischen Patentanmeldungen EP-A-244 380 und EP-A-247 983 beanspruchten Darreichungsformen wird für die säurelabilen Wirkstoffe eine Stabilisierung insbesondere durch den Zusatz von Basen zum Kern und somit eine Erhöhung des pH-Wertes erreicht; für die Erzielung einer ausreichenden Lagerstabilität müssen jedoch sowohl bei der Herstellung als auch bei der Lagerung bestimmte Bedingungen eingehalten werden, die mit einer optimalen galenischen Formulierung und einer problemlosen Vorratshaltung nur schlecht in Einklang zu bringen sind. So heißt es in der EP-A-247 983 sinngemäß: "Für die Langzeitstabilität bei der Lagerung ist es wesentlich, daß der Wassergehalt der den Wirkstoff Omeprazol enthaltenden Darreichungsform (magensaftresistent überzogene Tabletten, Kapseln und Pellets) niedrig gehalten wird und bevorzugt nicht mehr als 1,5 Gew.-% beträgt. Demzufolge sind Endverpackungen mit in Hartgelatinekapseln abgefüllten, magensaftresistent überzogenen Pellets bevorzugt mit Trockenmitteln zu versehen, die den Wassergehalt der Gelatinehüllen so weit senken, daß der Wassergehalt in den Pellets 1,5 Gew.-% nicht überschreitet".

Der bei der Herstellung von Pelletkernen aus Stabilitätsgründen niedrig zu haltende Wassergehalt bewirkt nun, daß die für die Pelletkernherstellung zu extrudierende Masse nicht ausreichend plastisch ist, um das Extrudat anschließend zu sphärischen Partikeln runden zu können. Es entstehen vielmehr zylindrische Körper, die bei den anschließenden Coating-Schritten an den Enden weniger dicke Lackschichten erhalten und somit an diesen Stellen nicht die geforderte Magensaftresistenz aufweisen und überdies den Kern nicht sicher von der magensaftresistenten Schicht durch ein Sub-coating schützen, was für die Stabilität wesentlich ist.

Die aufgezeigten Stabilitätsprobleme treten auch auf, wenn man versucht, den $H^+/K^+$-ATPase-Hemmer Pantoprazol (prop. INN für die Verbindung 5-(Difluormethoxy)-2-[(3,4-dimethoxy-2-pyridyl)methylsulfinyl]-1H-benzimidazol) so zu formulieren, wie dies in den europäischen Patentanmeldungen EP-A-244 380 und EP-A-247 983 beschrieben ist.

Beschreibung der Erfindung

Überraschenderweise wurde nun gefunden, daß beim Verzicht auf bestimmte, als Tablettenhilfsstoffe häufig verwendete Füllstoffe und Bindemittel, wie sie für die Herstellung der Pellet- bzw. Tablettenkerne in den europäischen Patentanmeldungen EP-A-244 380 und EP-A-247 983 angegeben sind, die geschilderten Stabilitätsprobleme nicht auftreten. Diese Füllstoffe bzw. Bindemittel sind insbesondere Lactose, mikrokristalline Zellulose und Hydroxypropylzellulose.

Gegenstand der Erfindung ist somit ein den Wirkstoff Pantoprazol enthaltendes, oral zu applizierendes, magensaftresistentes Arzneimittel in Pellet- oder Tablettenform, das aus einem basisch reagierenden Pellet- oder Tablettenkern, einer oder mehreren inerten, wasserlöslichen Zwischenschicht(en) und einer magensaftresistenten äußeren Schicht besteht, und das dadurch gekennzeichnet ist, daß der Kern neben Pantoprazol bzw. neben einem Pantoprazol-Salz als Bindemittel Polyvinylpyrrolidon und/oder Hydroxypropylmethylcellulose und gewünschtenfalls zusätzlich als inerten Füllstoff Mannit enthält.

Für eine basische Reaktion des Pellet- oder Tablettenkernes wird diesem - sofern die gewünschte Erhöhung des pH-Wertes nicht bereits durch Verwendung des Wirkstoff-Salzes erzielt wird - eine anorganische Base beigemischt. Hier seien beispielsweise die pharmakologisch verträglichen Alkali-, Erdalkali- oder Erdmetallsalze schwacher Säuren sowie die pharmakologisch verträglichen Hydroxide und Oxide von Erdalkali- und Erdmetallen genannt. Als beispielhaft hervorzuhebende Base sei Natriumcarbonat genannt.

Neben Füllstoff und Bindemittel kommen bei der Tablettenkernherstellung noch weitere Hilfsstoffe, insbesondere Gleit- und Trennmittel sowie Tabletten-Sprengmittel zum Einsatz.

Als Gleit- und Trennmittel seien beispielsweise Calciumsalze höherer Fettsäuren, wie z.B. Calciumstearat genannt.

Als Tabletten-Sprengmittel kommen insbesondere chemisch indifferente Mittel infrage. Als bevorzugtes Tabletten-Sprengmittel sei (quer)vernetztes Polyvinylpyrrolidon (z.B. Crospovidone) genannt.

Bezüglich der auf den Pellet- bzw. Tablettenkern aufzubringenden wasserlöslichen Zwischenschicht(en) wird auf solche wasserlöslichen Schichten verwiesen, wie sie üblicherweise vor der Aufbringung magensaftresistenter Schichten verwendet werden, oder wie sie z.B. in der DE-OS 39 01 151 beschrieben sind. Als für die Zwischenschicht verwendbare Filmpolymere seien beispielsweise Hydroxypropylmethylcellulose und/oder Polyvinylpyrrolidon genannt, denen gewünschtenfalls noch Weichmacher (wie etwa Propylenglykol) und/oder weitere Zusatz- und Hilfsstoffe (z.B. Puffer, Basen oder Pigmente) beigefügt werden können.

Welche magensaftresistenten äußeren Schichten verwendet werden können, ist dem Fachmann aufgrund seines Fachwissens bekannt. Vorteilhafterweise werden (zur Vermeidung organischer Lösungsmittel und da der erfindungsgemäße Kern nicht die aus dem Stand der Technik bekannte Wasserempfindlichkeit aufweist) wäßrige Dispersionen geeigneter magensaftresistenter Polymere, wie beispielsweise ein Methacrylsäure/Methacrylsäuremethylester-Copolymerisat, gewünschtenfalls unter Zusatz eines Weichmachers (z.B. Triethylacetat) verwendet.

Der Wirkstoff Pantoprazol ist bekannt aus dem europäischen Patent 166 287. Als Salze des Pantoprazols seien die im europäischen Patent 166 287 genannten Salze beispielhaft erwähnt. Ein bevorzugtes Salz ist das Natriumsalz.

Die Verwendung von Mannit als alleinigem Füllstoff für Tabletten erfordert ein geeignetes Bindemittel, das dem Kern eine ausreichende Härte verleihen muß. Bei dem für die Kern-Herstellung als Bindemittel verwendeten Polyvinylpyrrolidon handelt es sich insbesondere um ein Produkt mit höherem Molekulargewicht (ca. 300.000 bis 400.000). Als bevorzugtes Polyvinylpyrrolidon sei PVP 90 (Molekulargewicht ca. 360.000) genannt.

Die erfindungsgemäße orale Darreichungsform zeichnet sich gegenüber den aus dem Stand der Technik bekannten Darreichungsformen für andere $H^+/K^+$-ATPase-Hemmer mit Pyridylmethylsulfinyl-1H-benzimidazol-Struktur insbesondere dadurch aus, daß ein über 1,5 Gew.-% hinausgehender Wassergehalt im Tablettenkern nicht zu einer Verfärbung (Zersetzung) des Wirkstoffes führt. So werden auch bei einer höheren Restfeuchte im Granulat (von z.B. 5 bis 8 Gew.-%) stabile Tabletten erhalten.

Pellets können durch Auftragen einer Vorisolierung auf Saccharose-Starterpellets und anschließendes Auftragen einer 30 %igen isopropanolischen Wirkstofflösung mit Hydroxymethylpropylcellulose als Binder erhalten werden.

Der Auftrag der Isolierschicht kann analog zu den Tabletten auch unter Verwendung entsprechender Fertigdispersionen (z.B. Opadry) erfolgen. Der magensaftresistente Überzug erfolgt analog zu der Vorgehensweise bei Tabletten.

Weiterer Gegenstand der Erfindung ist ein Verfahren zur Herstellung der erfindungsgemäßen Arzneimittel in Pellet oder Tablettenform, das dadurch gekennzeichnet ist, daß man den erfindungsgemäßen Kern herstellt, mit einer oder mehreren inerten wasserlöslichen Zwischenschichten umgibt und eine magensaftresistente äußere Schicht aufträgt.

Die folgenden Formulierungsbeispiele erläutern die Erfindung näher, ohne sie einzuschränken.

**Beispiele**

**1. Tabletten**

I. Tablettenkern

| | |
|---|---|
| a) Pantoprazol-Na-Sesquihydrat | 45,1 mg |
| b) Natriumcarbonat | 10,0 mg |
| c) Mannit | 42,7 mg |
| d) Crospovidone | 50,0 mg |
| e) PVP 90 (Povidone) | 4,0 mg |
| f) Calciumstearat | 3,2 mg |
| | 155,0 mg |

a) wird mit einem Teil von b), c) und d) vermischt. Der Rest von b) und c) wird in die klare wässrige Lösung von e) gegeben und mit b) auf einen pH-Wert > 10 eingestellt. Mit dieser Lösung wird in der Wirbelschicht granuliert. Dem getrockneten Granulat wird der Rest von d) sowie f) zugesetzt und das Granulat auf einer geeigneten Tablettenmaschine verpreßt.

II. Vorisolierung (Zwischenschicht)

| | |
|---|---|
| g) HPMC 2910, 3cps | 15,83 mg |
| h) PVP 25 | 0,32 mg |
| i) Titandioxid | 0,28 mg |
| j) LB Eisenoxid-gelb 100 E 172 | 0,025 mg |
| k) Propylenglykol | 3,54 mg |
| | 20,00 mg |

| | |
|---|---|
| Gesamtgewicht pro vorisoliertem Kern | 175,00 mg |

g) wird in Wasser gelöst und h) zugegeben und ebenfalls gelöst (A). i) und j) werden mit einem geeigneten Rührer in Wasser suspendiert (B). A und B werden vereinigt. Nach Zugabe von k) wird die Suspension unmittelbar vor der weiteren Verarbeitung gesiebt, bei der die unter I. erhaltenen Tablettenkerne in einem geeigneten Gerät mit der Suspension in ausreichender Schichtdicke überzogen werden.

III. Magensaftresistenter Überzug

| | |
|---|---|
| l) Eudragit⁻ L 30 D | 13,64 mg |
| m) Triethylcitrat | 1,36 mg |
| | 15,00 mg |

| | |
|---|---|
| Gesamtgewicht pro magensaftresistenter Filmtablette | 190,00 mg |

l) wird mit Wasser verdünnt und m) zugesetzt. Die Dispersion wird vor der Verarbeitung gesiebt.
Auf die unter II. erhaltenen vorisolierten Kerne wird III. in geeigneten Apparaturen aufgesprüht.

**2. Pellets**

I. Starterpellets

| | |
|---|---|
| a) Saccharose Pellets (0,7-0,85 mm) | 950,0 g |
| b) Hydroxypropylmethylcellulose | 50,0 g |

a) wird mit der wäßrigen Lösung von b) in der Wirbelschicht (Wurster-Verfahren) besprüht.

II. Aktivpellets

| | |
|---|---|
| c) Pantoprazol-Na-Sesquihydrat | 403,0 g |
| d) Hydroxypropylmethylcellulose | 40,3 g |

c) und d) werden nacheinander in 30 % Isopropanol gelöst und auf 900 g der unter I. erhaltenen Starterpellets in der Wirbelschicht (Wurster-Verfahren) aufgesprüht.

III. Vorisolierung (Zwischenschicht)

Der Überzug erfolgt analog zu der bei den Tabletten beschriebenen Vorgehensweise im Kessel oder in der Wirbelschicht.

4

IV. Magensaftresistenter Überzug

Der Überzug erfolgt analog zu der bei den Tabletten beschriebenen Vorgehensweise im Kessel oder in der Wirbelschicht.

Anschließend werden die Pellets in Kapseln geeigneter Größe (z.B. 1) abgefüllt.

**Patentansprüche**

1.  Oral zu applizierendes, mangensaftresistentes Arzneimittel in Pellet- oder Tablettenform, bei dem die Pellets bzw. Tabletten aus
    - einem Kern, in dem der Wirkstoff oder dessen physiologisch verträgliches Salz im Gemisch mit einem oder mehreren Bindemitteln, Füllstoffen und gewünschtenfalls anderen Tablettenhilfsstoffen und gewünschtenfalls einer oder mehreren basisch reagierenden physiologisch verträglichen anorganischen Verbindungen vorliegt,
    - einer oder mehreren diesen Kern umgebenden inerten, wasserlöslichen Zwischenschichten und
    - einer magensaftresistenen äußeren Schicht bestehen,
    dadurch gekennzeichnet, daß im Kern als Wirkstoff Pantoprazol, als Bindemittel Polyvinylpyrrolidon und/oder Hydroxypropylmethylcellulose und gewünschtenfalls als Füllstoff Mannit verwendet wird.

2.  Arzneimittel nach Anspruch 1 in Tablettenform, dadurch gekennzeichnet, daß als Bindemittel Polyvinylpyrrolidon und/oder Hydroxypropylmethylcellulose und als Füllstoff Mannit verwendet wird.

3.  Arzneimittel nach Anspruch 1 in Pelletform, dadurch gekennzeichnt, daß als Bindemittel Polyvinylpyrrolidon und/oder Hydroxypropylmethylcellulose verwendet wird.

4.  Arzneimittel nach Anspruch 1 oder 2 oder 3, dadurch gekennzeichnet, daß als physiologisch verträgliches Wirkstoffsalz Pantoprazol-Natrium verwendet wird.

5.  Arzneimittel nach Anspruch 1 oder 2 oder 3, dadurch gekennzeichnet, daß als basisch reagierende, physiologisch verträgliche anorganische Verbindungen pharmakologisch verträgliche Alkali-, Erdalkali- oder Erdmetallsalze schwacher Säuren oder pharmakologisch verträgliche Hydroxide oder Oxide von Erdalkali- oder Erdmetallen verwendet werden.

6.  Arzneimittel nach Anspruch 1 oder 2 oder 3, dadurch gekennzeichnet, daß als basisch reagierende, physiologisch verträgliche anorganische Verbindung Natriumcarbonat verwendet wird.

Europäisches
Patentamt

**EUROPÄISCHER RECHERCHENBERICHT**

Nummer der Anmeldung

EP 92 11 0021

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 342 522 (EISAI)<br>* Ansprüche *<br>--- | 1-6 | A 61 K 9/20<br>A 61 K 9/24<br>A 61 K 9/28<br>A 61 K 9/32 |
| D,A | EP-A-0 247 983 (HÄSSLE)<br>* Ansprüche *<br>--- | 1-6 | A 61 K 9/54<br>A 61 K 31/44 |
| D,A | EP-A-0 244 380 (HÄSSLE)<br>* Ansprüche *<br>----- | 1-6 | |

|  | RECHERCHIERTE SACHGEBIETE (Int. Cl.5) |
|---|---|
|  | A 61 K |

Der vorliegende Recherchenbericht wurde für alle Patentansprüche erstellt

| Recherchenort | Abschlußdatum der Recherche | Prüfer |
|---|---|---|
| DEN HAAG | 11-08-1992 | SCARPONI U. |